# EUROPÄISCHE PATENTANMELDUNG

(11) **EP 1 905 417 A2**
(43) Veröffentlichungstag der Anmeldung: **02.04.2008**
(21) Anmeldenummer: 07020874.9
(22) Anmeldetag: 03.08.2005
(51) Int. Cl.: A61K 8/11, A61K 8/25, A61K 8/81, A61Q 17/04, C08K 3/36, C08K 9/02, C09D 7/12, B01F 17/00, B01F 17/52

(54) **Verwendung von statistischen Copolymeren**

(30) Priorität: 27.08.2004 DE 102004041536
(62) Teilanmeldung aus: 05769764.1
(71) Anmelder: Merck Patent GmbH, 64293 Darmstadt (DE)
(72) Erfinder: Koch, Matthias, Dr., 65183 Wiesbaden (DE); Anselmann, Ralf, Dr., 59348 Luedinghausen-Seppenrade (DE)

(57) **Zusammenfassung**

Die Erfindung betrifft die Verwendung von statistischen Copolymeren enthaltend mindestens eine Struktureinheit mit hydrophoben Resten und mindestens eine Struktureinheit mit hydrophilen Resten als Dispergiermittel zur Herstellung von Dispersionen mit inkompatibler disperser und kontinuierlicher Phase, insbesondere zur Dispergierung von Partikel mit hydrophiler Oberfläche in Ölen, Dispersionen bzw. Pulverzusammensetzungen, enthaltend statistische Copolymere und Partikel mit hydrophiler Oberfläche, sowie Verfahren zu deren Herstellung.

## Beschreibung

Die Erfindung betrifft die Verwendung von statistischen Copolymeren als Dispergiermittel zur Herstellung von Dispersionen mit inkompatibler disperser und kontinuierlicher Phase, insbesondere zur Dispergierung von Partikel mit hydrophiler Oberfläche in Ölen, Dispersionen bzw. Pulverzusammensetzungen, enthaltend statistische Copolymere und Partikel mit hydrophiler Oberfläche und Verfahren zu deren Herstellung.

Dispersionen finden in zahlreichen gebieten Anwendung. So sind Farben und Lacke, sowie kosmetische und pharmazeutische Zubereitungen, wie auch Reinigungs- und Beschichtungsmittel häufig Dispersionen von Partikeln in Flüssigkeiten. Ein wesentliches Problemfeld bei dem Einsatz von Dispersionen besteht in der Neigung von Partikeln Agglomerate zu bilden, wodurch die Lagerstabilität der Dispersionen beeinträchtigt werden kann. Daher besteht ständig Bedarf nach verbesserten Dispergiermitteln, die stabile Dispersionen gewährleisten.

Weiter ist es für die Stabilität von Dispersionen wichtig, dass disperse und kontinuierliche Phase kompatibel sind. Hydrophile Partikel lassen sich unter Einsatz von Dispergiermitteln vergleichsweise einfach in Wasser dispergieren, mit Ölen bilden sich jedoch nur schwer Dispersionen. Zur Kompatibilisierung derartiger nicht-kompatibler Phasen sind Dispergierhilfsmittel, die zwischen den Phasen vermitteln, üblich.

Allerdings sind teilweise sehr hohe Konzentrationen dieser Dispergierhilfsmittel erforderlich, um stabile Dispersionen zu erzeugen. Zur Herstellung stabiler Dispersionen von stark hydrophilen Partikeln in Ölen stehen bislang keine wirklich befriedigenden Dispergiermittel zu Verfügung.

Daher besteht weiterhin Bedarf nach Dispergiermitteln, die es erlauben stabile Dispersionen mit inkompatibler disperser und kontinuierlicher Phase, insbesondere Dispersionen hydrophiler Partikel in Ölen herzustellen.

Überraschend wurde jetzt gefunden, dass bestimmte Copolymere sich in hervorragender Weise als Dispergiermittel zur Herstellung von Dispersionen mit inkompatibler disperser und kontinuierlicher Phase eignen.

Ein erster Gegenstand der vorliegenden Erfindung ist daher die Verwendung von statistischen Copolymeren enthaltend mindestens eine Struktureinheit mit hydrophoben Resten und mindestens eine Struktureinheit mit hydrophilen Resten als Dispergiermittel zur Herstellung von Dispersionen mit inkompatibler disperser und kontinuierlicher Phase.

Insbesondere bevorzugt ist dabei Verwendung von statistischen Copolymeren enthaltend mindestens eine Struktureinheit mit hydrophoben Resten und mindestens eine Struktureinheit mit hydrophilen Resten als Dispergiermittel zur Dispergierung von Partikeln mit hydrophiler Oberfläche in Ölen.

Ein weiterer Gegenstand der vorliegenden Erfindung sind dementsprechend ölige Dispersion enthaltend hydrophile Partikel, dadurch gekennzeichnet, dass als Dispergiermittel mindestens ein statistisches Copolymer enthaltend mindestens eine Struktureinheit mit hydrophoben Resten und mindestens eine Struktureinheit mit hydrophilen Resten enthalten ist.

Ein erfindungsgemäßes Verfahren zur Herstellung einer öligen Dispersion von hydrophilen Partikeln, ist dabei dadurch gekennzeichnet, dass statistische Copolymere enthaltend mindestens eine Struktureinheit mit hydrophoben Resten und mindestens eine Struktureinheit mit hydrophilen Resten mit einem Öl und hydrophilen Partikeln vermischt werden. In einer bevorzugten Variante werden die statistischen Copolymere in einem Öl vorgelegt und anschließend die hydrophilen Partikel zugegeben. In einer anderen bevorzugten Variante werden eine wässrige Dispersion hydrophiler Partikel mit einer Lösung eines statistischen Copolymeren in einem hydrophoben Lösungsmittel gemischt (emulgiert) und anschließend das Wasser oder beide Lösungsmittel entfernt.

Ein erfindungsgemäßes Verfahren zur Herstellung einer wässrigen Dispersion von hydrophoben Partikeln, ist dadurch gekennzeichnet, dass statistische Copolymere enthaltend mindestens eine Struktureinheit mit hydrophoben Resten und mindestens eine Struktureinheit mit hydrophilen Resten mit Wasser und hydrophoben Partikeln vermischt werden. Dabei werden vorzugsweise die statistischen Copolymere in Wasser vorgelegt werden, und anschließend die hydrophoben Partikel zugegeben.

Dabei kann die Herstellung der Dispersionen, insbesondere von öligen Dispersionen, in einer bevorzugten Ausführungsform der vorliegenden Erfindung ausgehend von redispergierbaren Partikeln erfolgen. Entsprechende Pulverzusammensetzungen enthaltend hydrophile Partikel, dadurch gekennzeichnet, dass die hydrophilen Partikel mit mindestens einem statistischen Copolymeren enthaltend mindestens eine Struktureinheit mit hydrophoben Resten und mindestens eine Struktureinheit mit hydrophilen Resten beschichtet sind, sind daher ebenfalls ein Gegenstand der vorliegenden Erfindung.

Die Pulverzusammensetzungen können erhalten werden, indem eine Dispersion nach den oben angegebenen Verfahren hergestellt wird und anschließend das Lösungsmittel entfernt wird.

In den erfindungsgemäßen Pulverzusammensetzungen sind Partikel mit hydrophiler Oberfläche üblicherweise in einem Gewichtsanteil von 20 bis 95 Gew.-%, vorzugsweise 30 bis 80 Gew.-%, bezogen auf die Pulverzusammensetzung enthalten.

Durch die Auswahl von statistischen Copolymeren aus mindestens einem Monomer mit hydrophoben Resten und mindestens einem Monomer mit hydrophilen Resten ist es jetzt gelungen Dispergiermittel zu Verfügung zu stellen, welche das Dispergieren von Partikeln mit hydrophiler Oberfläche in hydrophoben Medien und umgekehrt ermöglichen. Gleichzeitig gelingt es durch die Verwendung dieser neuen Dispergiermittel die Partikel nahezu Agglomerat-frei als redispergierbare Pulverzusammensetzung aus den Dispersionen zu isolieren, da die individuellen Partikel sich unmittelbar Polymerbeschichtet abtrennen lassen.

Die mit Hilfe der statistischen Copolymere herstellbaren Dispersionen zeichnen sich durch eine ausgezeichnete Stabilität aus. Zusätzlich genügen oft vergleichsweise geringe Mengen der Copolymere zur Herstellung stabiler Dispersionen.

Darüberhinaus lassen sich die mit dieser Methode erhältlichen Pulverzusammensetzungen besonders einfach und gleichmäßig redispergieren.

Vorteilhaft wirkt sich weiterhin aus, dass sich in den erfindungsgemäßen Dispersionen wenig oder keine Agglomerate der dispergierten Partikel bilden. Insbesondere eine unerwünschte Beeinträchtigung der Transparenz solcher Dispersionen im sichtbaren Licht kann, wenn entsprechend kleine Partikel dispergiert werden, weitgehend vermieden werden.

Vorzugsweise weisen die dispergierten Partikel eine mittlere Teilchengröße bestimmt mittels dynamischer Lichtstreuung bzw. Transmisionselektronenmikroskop von 3 bis 200 nm, insbesondere von 20 bis 80 nm und ganz besonders bevorzugt von 30 bis 50 nm auf. In speziellen ebenfalls bevorzugten Ausführungsformen der vorliegenden Erfindung ist die Verteilung der Teilchengrößen eng, d.h. die Schwankungsbreite beträgt weniger als 100 % des Mittelwertes, insbesondere bevorzugt maximal 50 % des Mittelwertes.

In einer weiteren Variante der vorliegenden Erfindung weisen die dispergierten Partikel eine mittlere Teilchengröße im Bereich von 500 bis 5000 nm auf. Ebenso kann es erfindungsgemäß bevorzugt sein, anisotrope Teilchen, insbesondere bevorzugt Plättchen mit einer Dicke im Bereich von 500 bis 5000 nm und einem Durchmesser im Bereich von 5 bis 100 µm zu dispersgieren.

Insbesondere vorteilhaft ist der Einsatz der erfindungsgemäßen Dispergiermittel zur Dispergierung von hydrophilen Partikeln, die eine Metall(hydr)oxid-Oberfläche aufweisen, wobei das Metall(hydr)oxid vorzugsweise gewählt ist aus Oxiden bzw. Hydroxiden von Silicium, Aluminium, Magnesium, Antimon, Cer, Cobalt, Chrom, Indium, Nickel, Zink, Titan, Eisen, Yttrium, Zinn, Zirconium und Mischungen davon. Derartige Partikel sind nur sehr schwer durch Öle zu benetzen und gelten daher als mit üblichen Dispergiermitteln nur schwierig in Ölen zu dispergieren. Erfindungsgemäß insbesondere bevorzugt ist es dabei, wenn Silica-Partikel oder mit Silica beschichtete Partikel dispergiert werden.

So können vorzugsweise Siliciumdioxid-Partikel eingesetzt werden, die beispielsweise nach dem in US 4 911 903 beschriebenen Verfahren erhalten werden können. Die Kerne werden dabei durch hydrolytische Polykondensation von Tetraalkoxysilanen in einem wäßrigammoniakalischen Medium hergestellt, wobei man zunächst ein Sol von Primärteilchen erzeugt und anschließend durch ein kontinuierliches, kontrolliertes Zudosieren von Tetraalkoxysilan die erhaltenen SiO₂-Partikel auf die gewünschte Teilchengröße bringt. Mit diesem Verfahren sind monodisperse SiO₂-Kerne mit mittleren Teilchendurchmessern zwischen 0,05 und 10 µm bei einer Standardabweichung von 5 % herstellbar. Entsprechende Produkte befinden sich unter dem Handelsnamen Monospher® (Fa. Merck) im Handel.

Weiterhin können SiO₂-Partikel eingesetzt werden, die mit (Halb)Metallen oder im sichtbaren Bereich nichtabsorbierenden Metalloxiden, wie z.B. TiO₂, ZrO₂**,** ZnO₂, SnO₂ oder Al₂O₃, beschichtet sind. Die Herstellung von mit Metalloxiden beschichteter SiO₂-Kerne ist beispielsweise in US 5 846 310, DE 198 42 134 und DE 199 29 109 näher beschrieben.

So können auch monodisperse Partikel aus nichtabsorbierenden Metalloxiden wie TiO₂, ZrO₂, ZnO₂, SnO₂ oder Al₂O₃ oder Metalloxidgemischen eingesetzt werden. Ihre Herstellung ist beispielsweise in EP 0 644 914 beschrieben. Weiterhin ist das Verfahren gemäß EP 0 216 278 zur Herstellung monodisperser SiO₂-Partikel ohne weiteres und mit gleichem Ergebnis auf andere Oxide übertragbar. Zu einem Gemisch aus Alkohol, Wasser und Ammoniak, dessen Temperatur mit einem Thermostaten auf 30 bis 40 °C genau eingestellt wird, werden unter intensiver Durchmischung Tetraethoxysilan, Tetrabutoxytitan, Tetrapropoxyzirkon oder deren Gemische in einem Guss zugegeben und die erhaltene Mischung für weitere 20 Sekunden intensiv gerührt, wobei sich eine Suspension von monodispersen Partikeln im Nanometerbereich ausbildet. Nach einer Nachreaktionszeit von 1 bis 2 Stunden werden die Kerne auf die übliche Weise, z.B. durch Zentrifugieren, abgetrennt, gewaschen und getrocknet.

Andere Silica-Partikel, die ebenfalls nach der in der vorliegenden Erfindung beschriebenen Methode vorteilhaft dispergiert werden können sind beispielsweise unter den Handelsnamen Ronaspher® (Fa. Merck) oder Aerosil® (Fa. Degussa) kommerziell erhältlich. Allgemein können mit den erfindungsgemäß einzusetzenden Polymeren Silica-Partikel nahezu jeder Gestaltung dispergiert werden. So können die Partikel z.B. kugelförmig, hohl, plättchenförmig porös oder unporös, stäbchenförmig, plättchenförmig oder amorph und damit ohne spezielle geometrische Raumform gestaltet sein.

Entsprechende Partikel können beispielsweise als Füllmaterialien oder zur Beschichtung eingesetzt werden.

Weiter kann es sich in einer bevorzugten Ausführungsform der vorliegenden Erfindung bei den zu dispergierenden Partikeln um Kapseln handeln. Erfindungsgemäß besonders bevorzugt einzusetzende Kapseln weisen Wände auf, die durch einen SolGel-Prozeß, wie er in den Anmeldungen WO 00/09652, WO 00/72806 und WO 00/71084 beschrieben ist, erhalten werden können. Bevorzugt sind hier wiederum Kapseln, deren Wände aus Kieselgel (Silica; undefiniertes Silicium-oxid-hydroxid) aufgebaut sind. Die Herstellung entsprechender Kapseln ist dem Fachmann beispielsweise aus den zitierten Patentanmeldungen bekannt, deren Inhalt ausdrücklich auch zum Gegenstand der vorliegenden Anmeldung gehört. Besonders bevorzugt sind dabei Kapselen, die UV Filter enthalten. Sowohl für UVA wie auch UVB-Filter gibt es viele aus der Fachliteratur bekannte und bewährte Substanzen, z.B. Benzylidenkampferderivate wie 3-(4'-Methylbenzyliden)-dl-kampfer (z.B. Eusolex® 6300), 3-Benzylidenkampfer (z.B. Mexoryl® SD), Polymere von N-{(2 und 4)-[(2-oxoborn-3-yliden)methyl]benzyl}-acrylamid (z.B. Mexoryl® SW), N,N,N-Trimethyl-4-(2-oxoborn-3-ylidenmethyl)anilinium methylsulfat (z.B. Mexoryl® SK) oder (2-Oxoborn-3-yliden)toluoi-4-sulfonsäure (z.B. Mexoryl® SL), Benzoyl- oder Dibenzoylmethane wie 1-(4-tert-Butylphenyl)-3-(4-methoxyphenyl)propan-1,3-dion (z.B. Eusolex® 9020) oder 4-Isopropyldibenzoylmethan (z.B. Eusolex® 8020), Benzophenone wie 2-Hydroxy-4-methoxybenzophenon (z.B. Eusolex® 4360) oder 2-Hydroxy-4-methoxybenzophenon-5-sulfonsäure und ihr Natriumsalz (z.B. Uvinul® MS-40), Methoxyzimtsäureester wie Methoxyzimtsäureoctylester (z.B. Eusolex® 2292), 4-Methoxyzimtsäureisopentylester, z.B. als Gemisch der Isomere (z.B. Neo Heliopan® E 1000), Salicylatderivate wie 2-Ethylhexylsalicylat (z.B. Eusolex® OS), 4-Isopropylbenzylsalicylat (z.B. Megasol®) oder 3,3,5-Trimethylcyclohexylsalicylat (z.B. Eusolex® HMS), 4-Aminobenzoesäure und Derivate wie 4-Aminobenzoesäure, 4-(Dimethylamino)benzoesäure-2-ethylhexylester (z.B. Eusolex® 6007), ethoxylierter 4-Aminobenzoesäureethylester (z.B. Uvinul® P25), Phenylbenzimidazolsulfonsäuren, wie 2-Phenylbenzimidazol-5-sulfonsäure sowie ihre Kalium-, Natrium- und Triethanolaminsalze (z.B. Eusolex® 232), 2,2-(1,4-Phenylen)-bisbenzimidazol-4,6-disulfonsäure bzw. deren Salze (z.B. Neoheliopan® AP) oder 2,2-(1,4-Phenylen)-bisbenzimidazol-6-sulfonsäure;
und weitere Substanzen wie
- 2-Cyano-3,3-diphenylacrylsäure-2-ethylhexylester (z.B. Eusolex® OCR),
- 3,3'-(1,4-Phenylendimethylen)-bis-(7,7-dimethyl-2-oxobicyclo-[2.2.1]hept-1-ylmethansulfonsäure sowie ihre Salze (z.B. Mexoryl® SX) und
- 2,4,6-Trianilino-(p-carbo-2'-ethylhexyl-1'-oxi)-1,3,5-triazin ( z.B. Uvinul® T 150)
- 2-(4-Diethylamino-2-hydroxy-benzoyl)-benzoesäure hexylester (z.B. Uvinul®UVA Plus, Fa. BASF).
Die in der Liste aufgeführten Verbindungen sind nur als Beispiele aufzufassen. Selbstverständlich können in den Kapseln auch andere UV-Filter verwendet werden. Bevorzugte Verbindungen mit UV-filternden Eigenschaften sind 3-(4'-Methylbenzyliden)-dl-kampfer, 1-(4-tert-Butylphenyl)-3-(4-methoxy-phenyl)-pro-pan-1,3-dion, 4-Isopropyldibenzoylmethan, 2-Hydroxy-4-methoxy---ben-zo--phenon, Methoxyzimtsäureoctylester, 3,3,5-Trimethyl-cyclo-hexyl-sali-cylat, 4-(Dimethylamino)benzoesäure-2-ethyl-hexylester, 2-Cyano-3,3-di-phenylacrylsäure-2-ethylhexylester, 2-Phenyl-benzimidazol-5-sulfon-säure sowie ihre Kalium-, Natrium- und Triethanol-aminsalze.
Im Handel ist beispielsweise unter dem Namen Eusolex®UV Pearl™OMC von der Merck KGaA eine SiO₂-Kapsel erhältlich, die Methoxyzimtsäureoctylester als UV Filter enthält.

Weiterhin kann es erfindungsgemäß bevorzugt sein, anorganische UV-Filter zu dispergieren. Als anorganische UV-Filter werden dabei vorzugsweise nanopartikuläre Metalloxide für die erfindungsgemäße Verwendung eingesetzt. So eignen sich insbesondere Titandioxid, Eisenoxide, Zinkoxid oder auch Ceroxide zum Einsatz als UV Filter, wobei Titandioxid als Metalloxid erfindungsgemäß insbesondere bevorzugt ist, da es die erfindungsgemäßen Aufgaben in besonderer Weise erfüllt. Titandioxid kann dabei in Form von Rutil oder Anatas oder in amorpher Form, vorzugsweise aber in Form von Rutil und/oder Anatas, vorliegen. Bevorzugte Primärpartikelgröße liegt im Bereich vom 5 bis 50 nm. Dabei sind die Primärpartikel insbesondere bei Anatas vorzugsweise rund, während Rutil-Primärpartikel häufig in Nadel- oder Spindelform bis hin zu Ovalen ("eiförmig") auftreten. Es können erfindungsgemäß jedoch auch runde Rutil-Primärpartikel eingesetzt werden. Bevorzugte anorganische UV Filter weisen dabei eine Siliciumdioxid-Beschichtung auf, die das nanopartikulären Metalloxid möglichst vollständig bedeckt. Es hat sich gezeigt, dass es vorteilhaft ist, wenn der Siliciumdioxidgehalt bezogen auf das gesamte nanopartikuläre UV-Schutzmittel 5 bis 50 Gew.-%, vorzugsweise 8 bis 30 Gew.-% und insbesondere bevorzugt 12 bis 20 Gew.-% beträgt. Das resultierende nanopartikuläre UV-Schutzmittel zeigt üblicherweise eine Partikelgröße nach der Scherrer-Methode im Bereich von 5 nm bis 100 nm, vorzugsweise im Bereich 8 bis 50 nm und insbesondere bevorzugt unterhalb von 25 nm. Die im Transmissionselektronenmikroskop ermittelbaren Abmessungen des nanopartikulären UV-Schutzmittels liegen üblicherweise bei einer Länge von 5 bis 160 nm und einer Breite von 10 bis 70 nm. Vorzugsweise liegt die Länge im Bereich von 30 bis 70 nm und die Breite im Bereich von 18 bis 40 nm. Diese anorganischen UV-Filter werden in der Regel in einer Menge von 0,5 bis 20 Gewichtsprozent, vorzugsweise 2 - 10 %, in kosmetische Zubereitungen eingearbeitet. Insbesondere bevorzugt ist erfindungsgemäß die Verwendung der Copolymere zur Dispergierung von Silica-beschichtetem Titandioxid, das beispielsweise unter dem Namen Eusolex® T-AVO (Fa. Merck KGaA) im Handel erhältlich ist.

Die Partikel mit hydrophiler Oberfläche werden dabei üblicherweise in einem Gewichtsanteil von 1 bis 90 Gew.-%, vorzugsweise 10 bis 60 Gew.-%, bezogen auf die Dispersion, dispergiert.

Das Dispergiermittel wird üblicherweise in einer Konzentration von 0,5 bis 80 Gew.-%, vorzugsweise in einer Konzentration von 1 bis 50 Gew.-% und insbesondere bevorzugt in einer Konzentration von 2 bis 8 Gew.-%, bezogen auf die gesamte Dispersion, eingesetzt.

Die erfindungsgemäß bevorzugt einzusetzenden statistischen Copolymere zeigen dabei ein Gewichtsverhältnis von Struktureinheiten mit hydrophoben Resten zu Struktureinheiten mit hydrophilen Resten in den statistischen Copolymeren im Bereich 1:10 bis 500:1, vorzugsweise im Bereich 1:2 bis 100:1 und insbesondere bevorzugt im Bereich 1:1 bis 10:1 liegt. Das gewichtsmittlere Molgewicht der bevorzugten statistischen Copolymere liegt im Bereich von M_{w}=1000 bis 1 000 000 g/mol, vorzugsweise im Bereich von 2 000 bis 50 000 g/mol.

Es hat sich dabei gezeigt, dass insbesondere Copolymere, welche der Formel I entsprechen, wobei 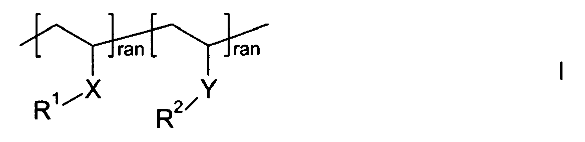 X und Y den Resten üblicher nichtionischer oder ionischer Monomere entsprechen und
R¹ steht für Wasserstoff oder eine hydrophobe Seitengruppe, vorzugsweise ausgewählt aus den verzweigten oder unverzweigten Alkylresten mit mindestens 4 Kohlenstoffatomen bei denen ein oder mehrere, vorzugsweise alle H-Atome durch Fluor-Atome ersetzt sein können, und
R² steht für eine hydrophile Seitengruppe, die vorzugsweise einen oder mehrere Phosphonat-, Phosphat-, Phosphonium-, Sulfonat-, Sulfonium-, (quartären) Amin-, Polyol- oder Polyether-Reste, besonders bevorzugt einen oder mehrere Hydroxylreste aufweist,
ran bedeutet, dass die jeweiligen Gruppen im Polymer statistisch verteilt angeordnet sind,
und wobei innerhalb eines Moleküls -X-R¹ und -Y-R² jeweils mehrere verschiedene Bedeutungen haben können und die Copolymere neben den in Formel I gezeigten Struktureinheiten weitere Struktureinheiten, vorzugsweise solche ohne oder mit kurzen Seitenketten, wie beispielsweise C₁₋₄-Alkyl enthalten können, die erfindungsgemäßen Anforderungen in besonderer Weise erfüllen.

Dabei sind solche Polymere gemäß Formel 1 wiederum besonders bevorzugt, bei denen X und Y unabhängig voneinander stehen für -O-, - C(=O)-O-, -C(=O)-NH-, -(CH₂)ₙ-, Phenyl, Naphthyl oder Pyridiyl. Weiter lassen sich Polymere bei denen mindestens eine Struktureinheit mindestens ein quarternäres Stickstoff- oder Phosphoratom enthält, wobei R² vorzugsweise steht für eine Seitengruppe -(CH₂)ₘ-(N⁺(CH₃)₂)-(CH₂)ₙ-SO₃⁻ oder eine Seitengruppe -(CH₂)ₘ-(N⁺(CH₃)₂)-(CH₂)ₙ-PO₃²⁻,-(CH₂)ₘ-(N⁺(CH₃)₂)-(CH₂)ₙ-O-PO₃⁻ oder eine Seitengruppe -(CH₂)ₘ-(P⁺(CH₃)₂)-(CH₂)ₙ-SO₃-, wobei m steht für eine ganze Zahl aus dem Bereich von 1 bis 30, vorzugsweise aus dem Bereich 1 bis 6, insbesondere bevorzugt 2, und n steht für eine ganze Zahl aus dem Bereich von 1 bis 30, vorzugsweise aus dem Bereich 1 bis 8, insbesondere bevorzugt 3, vorteilhaft einsetzen.

Insbesondere bevorzugt einzusetzende statistische Copolymere lassen sich dabei entsprechend der in DE 10 2004 004 210.1 beschriebenen Methode, nach folgendem Schema herstellen:

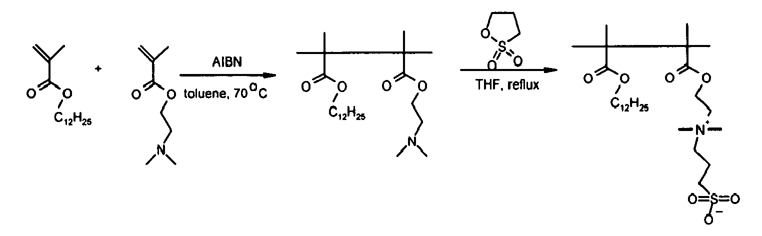

Dabei werden die gewünschten Mengen von Laurylmethacrylat (LMA) und Dimethylaminoethylmethacrylat (DMAEMA) nach bekannten Verfahren, vorzugsweise in Toluol radikalisch durch AIBN-Zusatz copolymerisiert. Anschließend wird eine Betainstruktur durch Umsetzung des Amins mit 1,3-Propansulton nach bekannten Methoden erhalten.
in einer insbesondere bevorzugten Variante wird ein Copolymeres aus Laurylmethacrylat (LMA) und Hydroxyethylmethacrylat (HEMA) eingesetzt. Auch diese Polymere wird vorzugsweise durch radikalische polymersiation der Monomere in Toluol durch AIBN-Zusatz copolymerisiert.

Alternative bevorzugt einzusetzende Copolymere können Styrol, Vinylpyrilidon, Vinylpyridin, halogeniertes Styrol oder Methoxystyrol enthalten, wobei diese Beispiele keine Einschränkung darstellen. In einer anderen ebenfalls bevorzugten Ausführungsform der vorliegenden Erfindung werden Polymere verwendet, die dadurch gekennzeichnet sind, dass mindestens eine Struktureinheit ein Oligo- oder Polymer, vorzugsweise ein Makromonomer ist, wobei Polyether, Polyolefine und Polyacrylate als Makromonomere insbesondere bevorzugt sind.

Es kann dabei erfindungsgemäß weiter bevorzugt sein, wenn die statistischen Copolymere mindestens eine Struktureinheit enthalten, die einen Phosphonium- oder Sulfonium-Rest aufweist.

Weiter kann es erfindungsgemäß bevorzugt sein, wenn in den statistischen Copolymeren neben der mindestens einen Struktureinheit mit hydrophoben Resten und der mindestens einen Struktureinheit mit hydrophilen Resten weitere Struktureinheiten, vorzugsweise solche ohne hydrophile oder hydrophobe Seitenketten bzw. mit kurzen Seitenketten, wie beispielsweise C₁₋₄-Alkyl enthalten sind.

In bestimmten Fällen kann es hilfreich sein, wenn neben dem statistischen Copolymeren ein weiteres Dispergiermittel, vorzugsweise ein nicht-ionisches Tensid eingesetzt wird. Bevorzugte CoDispergiermittel sind gegebenenfalls ethoxylierte oder propoxylierte, längerkettige Alkanole oder Alkylphenole mit unterschiedlichen Ethoxylierungs- bzw. Propoxylierungsgraden (z. B. Addukte mit 0 bis 50 mol Alkylenoxid; im Handel erhältlich z.B. von der Fa. BASF unter dem Handelsnamen Lutensol^{®}).

Auch Dispergierhilfsmittel können vorteilhaft eingesetzt werden, wobei vorzugsweise wasserlösliche hochmolekulare organische Verbindungen mit polaren Gruppen, wie Polyvinylpyrrolidon, Copolymerisate aus Vinylpropionat oder -acetat und Vinylpyrrolidon, teilverseifte Copolymeriste aus einem Acrylester und Acrylnitril, Polyvinylalkohole mit unterschiedlichem Restacetat-Gehalt, Zelluloseether, Gelatine, Blockcopolymere, modifizierte Stärke, niedermolekulare, carbon- und/oder sulfonsäuregruppenhaltigen Polymerisate oder Mischungen dieser Stoffe verwendet werden.

Besonders bevorzugte Schutzkolloide sind Polyvinylalkohole mit einem Restacetat-Gehalt von unter 40, insbesondere 5 bis 39 Mol.-% und/oder Vinylpyrrolidon-/Vinylpropionat-Copolymere mit einem Vinylestergehalt von unter 35, insbesondere 5 bis 30 Gew.-%.

Die ölige Phase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z. B. Rizinusöl;
- Organische Lösungsmittel, wie gesättigte und ungesättigte, cyclische und/oder acyclische Kohlenwasserstoffverbindungen, die gegebenenfalls Heteroatome wie O, N. S und P enthalten können,
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettlkoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäure und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe lsopropylmyristat, Isopropylpalmitat, Isopropylstearat, lsopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexaldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann auch die Ölphase ferner einen Gehalt an cyclischen oder linearan Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Erfindunsgemäß bevorzugte Dispersionen finden Einsatz als bzw. sind Farben oder Lacke, kosmetische oder pharmazeutische Zubereitungen oder Reinigungs- oder Beschichtungsmittel.

So sind kosmetische Zubereitungen, die Silicapartikel und/oder Silica-beschichtete partikuläre UV Filter und/oder Silica-verkaspelte UV-Filter enthalten ein bevorzugter Gegenstand der vorliegenden Erfindung. Die entsprechenden Materialien wurden bereits oben beschrieben.

Andere ebenfindungsgemäß ebenfalls bevorzugte Dispersionen sind mit Infrarot-Strahlung härtbare Lacke, die Antimon-Zinn-Oxid-Partikel enthalten. Beispiele für derartige Partikel sind die unter dem Handelnamen Minatec (Fa. Merck) vertriebenen Produkte.

Als Dispergiermedium kommen hier unter anderem Polymere, insbesondere Thermoplasten, wie PE, PP, PVC, PMMA, PS, ABS, Polyester, Polyamide in Frage. Die Dispergierung kann vorteilhaft über thermische Verfahren (extrudieren, kneten) oder unter Einsatz von Lösungen dieser Polymere in geeigneten Lösungsmitteln erfolgen.

Die nachfolgenden Beispiele sollen die Erfindung näher erläutern, ohne sie zu begrenzen.

### Beispiele

### Beispiel 1: Synthese eines statistischen Copolymers aus Dodecyl Methacrylat (Laurylmethacrylat; LMA) und Hydroxyethylmethacrylat (HEMA).

Die Kontrolle des Molekulargewichts kann erreicht werden durch Zugabe von Mercaptoethanol.

Es werden LMA und HEMA, in einer Menge entsprechend unten stehender Tabelle 1, in 12 g Toluol und 300 mg Mercaptoethanol vorgelegt und unter Argon bei 70°C nach Reaktionsstart durch Zugabe von 100mg AIBN in 1 mL Toluol für 18h radikalisch polymerisiert. Durch eine Nachinitiierung mit weiteren 50mg AIBN in 1 mL Toluol und weitere Reaktion für 12h wird bisher nicht umgesetztes Restmonomer ebenfalls Polymerisiert. Danach wird das Lösungsmittel bei vermindertem Druck entfernt und das erhaltene Polymer getrocknet. Die Charakterisierung der resultierenden Polymere findet sich in Tabelle 1.

**Tabelle 1: Eingesetzte Mengen an Monomeren und Charakterisierung der erhaltenen Polymere**

| | LMA [g] | HEMA [g] | M_{w} [g/mol] |
|---|---|---|---|
| E1 | 2,5 | 1,3 | 5800 |
| E2 | 3,8 | 1,3 | 5400 |
| E3 | 2,5 | 0,7 | 5500 |

### Beispiel 2: Dispergierung von SiO₂-Partikeln

800 mg des Polymeren aus Beispiel E2 werden in 20 g Paraffinöl gelöst. Beim Eintrag von 10 g Silicapartikel (Monospher 1000, Fa. Merck; Durchschnittliche Partikelgröße 1 µm) unter Rühren (2-Blatt-Rüherer; 200/min; kein nennenswerter Viskositätsanstieg) entsteht eine stabile Dispersion.

### Beispiel 3: Dispergierung von SiO₂-beschichteten TiO₂-Partikeln

800 mg des Polymeren aus Beispiel E2 werden in 20 g kosmetischem Öl (Miglyol® 8810 N; Fa. Condea; INCI: Butylene Glycol DiCarprylate/Dicaprate) gelöst. Beim Eintrag von 10 g Eusolex® T-AVO (Fa. Merck) unter Rühren (2-Blatt-Rüherer; 200/min) entsteht eine stabile Dispersion.

### Beispiel 4: Dispergierung von SiO₂-beschichteten TiO₂-Partikeln

Eine Dispersion enthaltend
6 Gew.-% des Polymeren aus Beispiel E2
57 Gew.-% Miglyol 8810 N (Fa. Condea)
37 Gew.-% Eusolex® T-AVO (Fa. Merck)
wird mit einer Dispergierscheibe und anschließend für ca. 5 Minuten mit einem U-Turax (bei 8000 upm) homogenisiert. Nach einem Tag Standzeit liegt die Viskosität bei ca. 12500 mPa s.

### Beispiel 5: Dispergierung von Antimon-Zinn-Oxid-Partikeln

800 mg des Polymeren aus Beispiel E2 werden in 20 g Terpineol gelöst. Beim Eintrag von 20 g Antimon-Zinn-Oxid-Partikel (Minatec® , Fa. Merck) unter Rühren (2-Blatt-Rüherer; 200/min; kein nennenswerter Viskositätsanstieg) entsteht eine stabile Dispersion.

### Beispiel 6: Herstellung redispergierbarer, nanoskaliger Antimon-Zinn-Oxid-Partikel

1g des Polymeren aus Beispiel E2 werden in 100 g Toluol gelöst. Darin werden 10g einer stabilen wäßrigen Dispersion von Antimon-Zinn-Oxid-Partikeln (Minatec®, Fa. Merck), Feststoffgehalt 2g, emulgiert (U-Turrax, Ultraschall). Das Lösungsmittelgemisch wird entfernt. Man erhält hydrophobisierte Partikel, die sich in organischen Lösungsmitteln (z.B. Toluol) sehr leicht redispergieren lassen.

### Beispiel 7: Dispergierung von pyrogener Kieselsäure

800 mg des Polymeren aus Beispiel E2 werden in 20 g Toluol gelöst. Beim Eintrag von 10 g pyrogener Kieselsäure (Aerosil^{®} 500X; Fa. Degussa) unter Rühren (2-Blatt-Rüherer; 200/min; kein nennenswerter Viskositätsanstieg) entsteht eine stabile Dispersion.

## Patentansprüche

1. Verwendung von statistischen Copolymeren, enthaltend mindestens eine Struktureinheit mit hydrophoben Resten und mindestens eine Struktureinheit mit hydrophilen Resten als Dispergiermittel zur Dispergierung von Partikeln mit hydrophiler Oberfläche in Ölen, wobei es sich bei den Partikeln um Silica-Partikel oder mit Silica beschichtete Partikel handelt.

2. Verwendung nach Anspruch 1, **dadurch gekennzeichnet, dass** die hydrophilen Partikel eine Metall(hydr)oxid-Oberfläche aufweisen, wobei das Metall(hydr)oxid vorzugsweise gewählt ist aus Oxiden bzw. Hydroxiden von Silicium, Aluminium, Magnesium, Antimon, Cer, Cobalt, Chrom, Indium, Nickel, Zink, Titan, Eisen, Yttrium, Zinn, Zirconium und Mischungen davon.

3. Verwendung nach mindestens einem der vorstehenden Ansprüche, **dadurch gekennzeichnet, dass** das Dispergiermittel in einer Konzentration von 0,5 bis 80 Gew.-%, vorzugsweise in einer Konzentration von 1 bis 50 Gew.-% und insbesondere bevorzugt in einer Konzentration von 2 bis 8 Gew.-%, bezogen auf die gesamte Dispersion, eingesetzt wird.

4. Verwendung nach mindestens einem der vorstehenden Ansprüche, **dadurch gekennzeichnet, dass** die Partikel mit hydrophiler Oberfläche in einem Gewichtsanteil von 1 bis 90 Gew.-%, vorzugsweise 10 bis 60 Gew.-%, bezogen auf die Dispersion, dispergiert werden.

5. Verwendung nach mindestens einem der vorstehenden Ansprüche, **dadurch gekennzeichnet, dass** mindestens ein weiteres Dispergiermittel und/oder Dispergierhilfsmittel eingesetzt wird.

6. Verwendung nach mindestens einem der vorstehenden Ansprüche, **dadurch gekennzeichnet, dass** das Gewichtsverhältnis von Struktureinheiten mit hydrophoben Resten zu Struktureinheiten mit hydrophilen Resten in den statistischen Copolymeren im Bereich 1:10 bis 500:1, vorzugsweise im Bereich 1:2 bis 100:1 und insbesondere bevorzugt im Bereich 1:1 bis 10:1 liegt.

7. Verwendung nach mindestens einem der vorstehenden Ansprüche, **dadurch gekennzeichnet, dass** das gewichtsmittlere Molgewicht der statistischen Copolymere im Bereich von M_{w}=1000 bis 1 000 000 g/mol, vorzugsweise im Bereich von 2 000 bis 50 000 g/mol liegt.

8. Verwendung nach mindestens einem der vorstehenden Ansprüche, **dadurch gekennzeichnet, dass** die Copolymere im wesentlichen der Formel I entsprechen, wobei 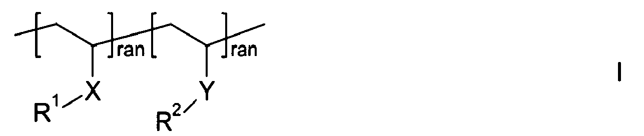 X und Y den Resten üblicher nichtionischer oder ionischer Monomere entsprechen und
R¹ steht für Wasserstoff oder eine hydrophobe Seitengruppe, vorzugsweise ausgewählt aus den verzweigten oder unverzweigten Alkylresten mit mindestens 4 Kohlenstoffatomen bei denen ein oder mehrere, vorzugsweise alle H-Atome durch Fluor-Atome ersetzt sein können, und
R² steht für eine hydrophile Seitengruppe, die vorzugsweise einen oder mehrere Phosphonat-, Phosphat-, Phosphonium-, Sulfonat-, Sulfonium-, (quartären) Amin-, Polyol- oder Polyether-Reste, besonders bevorzugt einen oder mehrere Hydroxylreste aufweist, ran bedeutet, dass die jeweiligen Gruppen im Polymer statistisch verteilt angeordnet sind,
und wobei innerhalb eines Moleküls -X-R¹ und -Y-R² jeweils mehrere verschiedene Bedeutungen haben können und die Copolymere neben den in Formel 1 gezeigten Struktureinheiten weitere Struktureinheiten, vorzugsweise solche ohne oder mit kurzen Seitenketten, wie beispielsweise C₁₋₄-Alkyl enthalten können.

9. Verwendung nach Anspruch 8, **dadurch gekennzeichnet, dass** X und Y unabhängig voneinander stehen für -O-, -C(=O)-O-, -C(=O)-NH-, -(CH₂)ₙ-, Phenylen oder Pyridiyl.

10. Verwendung nach mindestens einem der Ansprüche 8 oder 9, **dadurch gekennzeichnet, dass** mindestens eine Struktureinheit des Copolymeren mindestens ein quarternäres Stickstoff- oder Phospheratom enthält, wobei R² vorzugsweise steht für eine Seitengruppe -(CH₂)ₘ-(N⁺(CH₃)₂)-(CH₂)ₙ-SO₃⁻ oder eine Seitengruppe -(CH₂)ₘ-(N⁺(CH₃)₂)-(CH₂)ₙ-PO₃²⁻, -(CH₂)ₘ-(N⁺(CH₃)₂)-(CH₂)ₙ-O-PO₃²⁻ oder eine Seitengruppe -(CH₂)ₘ-(P⁺(CH₃)₂)-(CH₂)ₙ-SO₃-, wobei m steht für eine ganze Zahl aus dem Bereich von 1 bis 30, vorzugsweise aus dem Bereich 1 bis 6, insbesondere bevorzugt 2, und n steht für eine ganze Zahl aus dem Bereich von 1 bis 30, vorzugsweise aus dem Bereich 1 bis 8, insbesondere bevorzugt 3.

11. Verwendung nach mindestens einem der Ansprüche 1 bis 9, **dadurch gekennzeichnet**, das als statistisches Copolymeres ein Copolymer im wesentlichen bestehend aus Laurylmethacrylat (LMA) und Hydroxyethylmethacrylat (HEMA) eingesetzt wird.

12. Verwendung nach mindestens einem der Ansprüche 1 bis 10, **dadurch gekennzeichnet, dass** mindestens eine Struktureinheit des Copolymeren ein Oligo- oder Polymer, vorzugsweise ein Makromonomer ist, wobei Polyether, Polyolefine und Polyacrylate als Makromonomere insbesondere bevorzugt sind.

13. Verwendung nach mindestens einem der Ansprüche 1 bis 10 oder 12, **dadurch gekennzeichnet, dass** mindestens eine Struktureinheit des Copolymeren einen Phosphonium- oder Sulfonium-Rest aufweist.

14. Verwendung nach mindestens einem der vorstehenden Ansprüche, **dadurch gekennzeichnet, dass** in den statistischen Copolymeren neben der mindestens einen Struktureinheit mit hydrophoben Resten und der mindestens einen Struktureinheit mit hydrophilen Resten weitere Struktureinheiten, vorzugsweise solche ohne hydrophile oder hydrophobe Seitenketten bzw. mit kurzen Seitenketten, wie C₁₋₄-Alkyl enthalten sind.

15. Ölige Dispersion, die eine kosmetische Zubereitung ist, die Silicapartikel und/ oder Silica-beschichtete partikuläre UV Filter enthält, enthaltend hydrophile Partikel, **dadurch gekennzeichnet, dass** als Dispergiermittel mindestens ein statistisches Copolymer enthaltend mindestens eine Struktureinheit mit hydrophoben Resten und mindestens eine Struktureinheit mit hydrophilen Resten enthalten ist.

16. Dispersion nach Anspruch 15, **dadurch gekennzeichnet, dass** das Dispergiermittel in einer Konzentration von 0,5 bis 80 Gew.-%, vorzugsweise in einer Konzentration von 1 bis 50 Gew.-% und insbesondere bevorzugt in einer Konzentration von 2 bis 8 Gew.-%, bezogen auf die gesamte Dispersion, vorliegt.

17. Dispersion nach mindestens einem der vorstehenden Ansprüche, **dadurch gekennzeichnet, dass** das die hydrophilen Partikel eine Metall(hydr)oxid-Oberfläche aufweisen, wobei das Metall(hydr)oxid vorzugsweise gewählt ist aus Oxiden bzw. Hydroxiden von Silicium, Aluminium, Magnesium Antimon, Cer, Cobalt, Chrom, Indium, Nickel, Zink, Titan, Eisen, Yttrium, Zinn, Zirconium und Mischungen davon, wobei es sich bei den Partikeln insbesondere bevorzugt um Silica-Partikel oder mit Silica beschichtete Partikel handelt.

18. Dispersion nach mindestens einem der vorstehenden Ansprüche, **dadurch gekennzeichnet, dass** die Partikel mit hydrophiler Oberfläche in einem Gewichtsanteil von 1 bis 90 Gew.-%, vorzugsweise 10 bis 60 Gew.-%, bezogen auf die Dispersion, vorliegen.

19. Ölige Dispersion, die ein mit Infrarot-Strahlung härtbarer Lack ist, der Antimon-Zinn-Oxid-Partikel enthält, enthaltend hydrophile Partikel, **dadurch gekennzeichnet, dass** als Dispergiermittel mindestens ein statistisches Copolymer enthaltend mindestens eine Struktureinheit mit hydrophoben Resten und mindestens eine Struktureinheit mit hydrophilen Resten enthalten ist.

20. Dispersion nach Anspruch 17, **dadurch gekennzeichnet, dass** das Dispergiermittel in einer Konzentration von 0,5 bis 80 Gew.-%, vorzugsweise in einer Konzentration von 1 bis 50 Gew.-% und insbesondere bevorzugt in einer Konzentration von 2 bis 8 Gew.-%, bezogen auf die gesamte Dispersion, vorliegt.

21. Dispersion nach mindestens einem der vorstehenden Ansprüche, **dadurch gekennzeichnet, dass** die hydrophilen Partikel eine Metal(hydr)oxid-Oberfläche aufweisen, wobei das Metall(hydr(oxid vorzugsweise gewählt ist aus Oxiden bzw. Hydroxiden von Silicium, Aluminium, Magnesium, Antimon, Cer, Cobalt, Chrom, Indium, Nickel, Zink, Titan, Eisen, Yttrium, Zinn, Zirconium und Mischungen davon, wobei es sich bei den Partikeln insbesondere bevorzugt um Silica-Partikel handelt.

22. Dispersion nach mindestens einem der vorstehenden Ansprüche, **dadurch gekennzeichnet, dass** die Partikel mit hydrophiler Oberfläche in einem Gewichtsanteil von 1 bis 90 Gew.-%, vorzugsweise 10 bis 60 Gew.-%, bezogen auf die Dispersion vorliegen.

23. Verfahren zur Herstellung einer öligen Dispersion von hydrophilen Partikeln, **dadurch gekennzeichnet, dass** statistische Copolymere enthaltend mindestens eine Struktureinheit mit hydrophoben Resten und mindestens eine Struktureinheit mit hydrophilen Resten mit einem Öl und hydrophilen Partikeln vermischt werden und dass nach der Herstellung das Lösungsmittel entfernt wird.

24. Verfahren zur Herstellung einer öligen Dispersion nach Anspruch 23, **dadurch gekennzeichnet, dass** die statistischen Copolymere in einem Öl vorgelegt werden, und anschließend die hydrophilen Partikel zugegeben werden.

25. Verfahren zur Herstellung einer öligen Dispersion nach Anspruch 23, **dadurch gekennzeichnet, dass** eine wässrige Dispersion hydrophiler Partikel mit einer Lösung eines statistischen Copolymeren in einem hydrophoben Lösungsmittel gemischt (emulgiert) werden und das Wasser oder beide Lösungsmittel entfernt werden.

26. Verfahren zur Herstellung einer wässrigen Dispersion von hydrophoben Partikeln, **dadurch gekennzeichnet, dass** statistische Copolymere enthaltend mindestens eine Struktureinheit mit hydrophoben Resten und mindestens eine Struktureinheit mit hydrophilen Resten mit Wasser und hydrophoben Partikeln vermischt werden.

27. Verfahren zur Herstellung einer wässrigen Dispersion nach Anspruch 26, **dadurch gekennzeichnet, dass** die statistischen Copolymere in Wasser vorgelegt werden, und anschließend die hydrophoben Partikel zugegeben werden.

28. Pulverzusammensetzung enthaltend hydrophile Partikel, **dadurch gekennzeichnet, dass** die hydrophilen Partikel mit mindestens einem statistischen Copolymeren enthaltend mindestens eine Struktureinheit mit hydrophoben Resten und mindestens eine Struktureinheit mit hydrophilen Resten beschichtet sind.

29. Pulverzusammensetzung nach Anspruch 28, **dadurch gekennzeichnet; dass** das die hydrophilen Partikel eine Metall(hydr)oxid-Oberfläche aufweisen, wobei das Metall(hydr)oxid vorzugsweise gewählt ist aus Oxiden bzw. Hydroxiden von Silicium, Aluminium, Magnesium Antimon, Cer, Cobalt, Chrom, Indium, Nickel, Zink, Titan, Eisen, Yttrium, Zinn, Zirconium und Mischungen davon, wobei es sich bei den Partikeln insbesondere bevorzugt um Silica-Partikel oder mit Silica beschichtete Partikel handelt.

30. Pulverzusammensetzung nach mindestens einem der vorstehenden Ansprüche, **dadurch gekennzeichnet, dass** die Partikel mit hydrophiler Oberfläche in einem Gewichtsanteil von 20 bis 95 Gew.-%, vorzugsweise 30 bis 80 Gew.-%, bezogen auf die Pulverzusammensetzung vorliegen.

31. Pulverzusammensetzung nach mindestens einem der vorstehenden Ansprüche, **dadurch gekennzeichnet, dass** die hydrophilen Partikel im wesentlichen Silicapartikel und/oder Silica-beschichtete partikuläre UV Filter, insbesondere Silicabeschichtetes Titandioxid, sind.

32. Pulverzusammensetzung nach mindestens einem der Ansprüche 28 bis 30, **dadurch gekennzeichnet, dass** die hydrophilen Partikel im wesentlichen Antimon-Zinn-Oxid-Partikel sind.

33. Verfahren zur Herstellung einer Pulverzusammensetzung, **dadurch gekennzeichnet, dass** eine Dispersion gemäß mindestens einem der Ansprüche 26 bis 27 hergestellt wird und anschließend das Lösungsmittelentfernt wird.

34. Verfahren zur Herstellung einer Dispersion, **dadurch gekennzeichnet, dass** eine Pulverzusammensetzung gemäß mindestens einem der Ansprüche 28 bis 32 mit mindestens einem öligen Trägermaterial vermischt wird.
